# EUROPÄISCHE PATENTSCHRIFT

(11) **EP 0 955 017 B1**
(45) Veröffentlichungstag und Bekanntmachung des Hinweises auf die Patenterteilung: **07.06.2006**
(21) Anmeldenummer: 99107942.7
(22) Anmeldetag: 22.04.1999
(51) Int. Cl.: A61F 2/06

(54) **Radial aufweitbarer Stent**
Radially expandable stent
Stent radialement dilatable

(30) Priorität: 04.05.1998 DE 19819629
(43) Veröffentlichungstag der Anmeldung: 10.11.1999
(73) Patentinhaber: Abbott Laboratories Vascular Enterprises Limited, Dublin 2 (IE)
(72) Erfinder: Herrn Dr.-Ing. von Oepen Randolf, D-72145 Hirrlingen (DE)
(74) Vertreter: Schmitz, Hans-Werner

(56) Entgegenhaltungen:
- EP-A- 0 749 729
- EP-A- 0 775 472
- WO-A-93/22986
- US-A- 5 683 451
- US-A- 5 735 892

## Beschreibung

Die Erfindung betrifft eine Kombination, bestehend aus einer Vorrichtung zur Implantierung eines Stent-Graft in ein Körpergefäß und einem Stent-Graft, insbesondere in die Karotis, gemäss dem Oberbegriff des Anspruches 1. Eine derartige Vorrichtung ist aus der US-A-5683451 bekannt.

Stents in den Blutgefäßen haben die Aufgabe, eine erneute Verengung bzw. einen erneuten Verschluss der Blutgefäße nach einer Dilatation zu verhindern, um einen guten Durchfluss des Blutes wieder sicherzustellen. Stents in den Karotiden haben neben dieser Aufgabe auch die Aufgabe zu verhindern, dass sich Ablagerungen an den Blutgefäßen ablösen und in die Gehirngefäße gespült werden, damit der Patient keinen Gehirnschlag oder andere bleibende Schädigungen erleidet.

Konventionelle Stents, aufgebaut aus einer Zellen- bzw. Maschenstruktur, sind nur begrenzt in der Lage, Ablagerungen zurückzuhalten. Lösen sich diese Ablagerungen in den Karotiden, werden sie mit dem Blutstrom ins Gehirn gespült und können hier zur Verstopfung von Gefäßen führen. Eine Blockade in der Blutzufuhr löst in der Regel eine Gehirnschädigung in Form eines Gehirnschlages aus und ist daher unter allen Umständen zu vermeiden.

Stents werden in Blutgefäße eingesetzt, um die Möglichkeit einer erneuten Verengung zu reduzieren bzw. zu verhindern. Weiterhin werden so genannte Stent-Grafts eingesetzt, um Fehlstellen wie Aneurysmen, Rupturen, Dissektionen, Punkturen etc. zu überbrücken oder um eine Versiegelung des veränderten bzw. erkrankten Materials zu ermöglichen.

Stent-Grafts sind Stents, welche mit einem bioverträglichen Material überzogen sind und gegenüber Blut bzw. Partikeln, die sich von der Gefäßwand ablösen und in den Blutstrom geschwemmt werden können, undurchlässig sind.

Bei Stent-Grafts wird zwischen ballonexpandierbaren und selbstexpandierenden Systemen unterschieden. Ballonexpandierbare Systeme zeichnen sich dadurch aus, dass sie dem Gefäßdurchmesser genau angepasst werden können. Mit diesem System ist es möglich, der Verjüngung eines Gefäßes Rechnung zu tragen.

Selbstexpandierbare Stent-Grafts werden entweder aus geflochtenen, gewobenen oder gewirkten federnden Materialien aufgebaut oder es wird ein selbstexpandierbares Material, wie beispielsweise NITINOL verwendet. Der überzug besteht zumeist aus PETP (Dacron) oder PTFE (Teflon). PTFE zeichnet sich je nach Verarbeitung dadurch aus, dass es sich in eine Richtung dehnen lässt, ohne dass Rückstellkräfte auftreten. Insbesondere bei ballonexpandierbaren Systemen besteht daher die Möglichkeit, den Stent auf die gewünschte Dimension zu expandieren und genau dem Durchmesser des Gefäßes anzupassen.

Sofern notwendig ist es möglich, einen solchen Stent auch nach erfolgter Implantation gegebenenfalls mit einem Ballon nachzudehnen.

Stent-Grafts, welche mit PETP-Gewebe überzogen sind, können nur bis zu der Dimension gedehnt werden, bei der das Gewebe gespannt ist. Daher muss die Dimension des zu behandelnden Gefäßes vorher genau bestimmt werden. Eine Anpassung des Stent-Graft an die Anatomie des Gefäßes ist nur begrenzt möglich.

Selbstexpandierbare Systeme zeichnen sich durch die Besonderheit aus, dass sie bei einer Kompression von außen wieder in ihre ursprüngliche Lage zurückfedern.

Insbesondere bei Gefäßen, welche von außen ertastet werden oder durch benachbarte Muskeln deformiert werden können, werden bevorzugt selbstexpandierbare, federnde Systeme eingesetzt.

Die Erfindung soll eine Kombination, bestehend aus einer Vorrichtung und einem Stent-Graft der im Oberbegriff der Anspruches 1 argegebinen Gattung schoffer, die nicht nur Blutgefäße im aufgeweiteten Zustand hält, sondern die auch Ablagerungen an den Blutgefäßen hindert, sich abzulösen und in die Gehirngefäße gespült zu werden.

Die Aufgabe wird durch eine Kombination, bestehend aus einer Vorrichtung und einem Stent-Graft mit den Merkmalen des Anspruches 1 gelöst.

Die Erfindung zeichnet sich dadurch aus, dass ein selbstexpandierender Stent mit einem Material, beispielsweise PTFE, in der Form ummantelt wird, dass die Kraft der Selbstexpansion nicht ausreicht, die Ummantelung zu dehnen. Dieser Stent muss; ähnlich einem konventionellen ballonexpandierbaren Stent, durch einen Ballon aufgedehnt werden.

Die Besonderheit der Erfindung liegt darin, dass diese Vorrichtung und der Stent-Graft sich im Gegensatz zu einem ballonexpandierenden Stent-Graft zurück-stellt, sollte er durch äußere Kräfte verformt werden. Gleich-zeitig ist es aber möglich, den Vorteil von ballonexpandierbaren Vorrichtungen auszunützen und den Stent-Graft genau der Anatomie des Gefäßes anzupassen.

Wie bereits oben beschrieben, liegt einer der Vorteile von Stent-Grafts darin, dass es mit diesen Vorrichtungen möglich ist, Ablagerungen an der Gefäßwand zu fixieren und zu vermeiden, dass diese in den Blutstrom gelangen.

Ein ballonexpandierbarer Stent mit einer äußeren irreversiblen, dehnbaren Hülle, welcher zudem federnde Eigenschaften besitzt, eignet sich insbesondere für Einsätze in Gefäßen, die über der Länge der Stentung einen unterschiedlichen Durchmesser aufweisen. Der Durchmesser von Gefäßen kann um zwei und mehr Millimeter differieren, wie beispielsweise der übergang von der Carotis Communa in die Carotis Interna bzw. Externa.

Aktuelle selbstexpandierende Vorrichtungen sind nicht in der Lage, diese stark unterschiedlichen Durchmesser sinnvoll abzudecken. Entweder muss bei selbstexpandierbaren Stents der Stent so groß gewählt werden, dass eine gute Adaptierung an die Wand gewährleistet ist, oder der Stent wird passend für das kleinere Gefäß ausgewählt.

Im ersten Fall übt der Stent einen ständigen Druck auf die Gefäßwand des kleineren Gefäßes aus, was dazu führen kann, dass sich dieses Gefäß im Laufe der Zeit dehnt und der Geometrie des Stents anpasst. Dieser Effekt ist in jedem. Fall unerwünscht.

Wird ein selbstexpandierbarer Stent passend für das kleinere Gefäß ausgewählt, so besteht die Gefahr, dass der Stent im größeren Gefäß bzw. in der Region, in der das Gefäß einen größeren Durchmesser aufweist, nicht sauber an der Wand anliegt. Insbesondere bei Stent-Grafts besteht die Möglichkeit, dass Gebiete zwischen Stent-Graft und Gefäßwand entstehen, in denen es durch Verwirbelungen zu einer Thrombenbildung kommt, die in jedem Fall zu vermeiden ist.

Die beschriebene Erfindung bietet die Möglichkeit, bei einer möglichen Kompression durch die federnden Eigenschaften des Stent-Materials wieder auf den eingestellten Durchmesser zurückzufedern. Gleichzeitig garantiert diese Vorrichtung mit dem Stent-Graft die Möglichkeit, durch den Einsatz unterschiedlicher Ballondurchmesser den Stent der Anatomie des Gefäßes perfekt anzupassen.

Soll ein mit einem expandierbaren Material überzogener Stent in einer Gefäßverzweigung eingesetzt werden, so kann der Stent zumindest eine radiale öffnung aufweisen, welche im Gefäßsystem so positioniert werden kann, dass ein entsprechender Seitenast nicht verschlossen wird. Im Bereich dieser öffnung weist die Hülle eine nach der Expansion vorzugsweise kreisförmige öffnung auf, um den Blutstrom in dem Seitenast nicht zu beeinflussen.

Weitere Ausführungsformen des Stents können gegebenenfalls mehrere radiale öffnungen über der Länge des Stents aufweisen.

Die Hülle kann aus einem expandierbaren PTFE bestehen. Stents mit einer Hülle aus expandierbarem PTFE sind kostengünstig herzustellen. Wegen der guten Verträglichkeit mit dem Gewebe des Patienten kann die Hülle aber auch aus einem homologen Material bestehen. Hüllen aus einem tierischen Material, insbesondere aus Schweine-, Rinder- oder Pferdevenen, sind ebenfalls mit dem menschlichen Gewebe relativ gut verträglich. Das selbstexpandierbare Material des hohlzylindrischen Elements kann eine Nickel-Titan-Legierung (NITINOL), ein Federstahl oder ein polymerer Werkstoff sein.

## Patentansprüche

1. Kombination, bestehend aus einer Vorrichtung zur Implantierung eines Stent-Grafts in ein Körpergefäß und einem Stent-Graft
- mit einem selbstexpandierbaren hohlzylindrischen Stent;
- mit einer Hülle, die den Stent zur Bildung eines Stent-Grafts ummantelt; und
- mit einem expandierbaren Ballon zur Expansion des Stent-Grafts nach dem Einsetzen in das Körpergefäß;
**dadurch gekennzeichnet,**
- **dass** die Hülle aus irreversibel expandierbarem Material gefertigt ist und den Stent vor der Platzierung im Körpergefäß in seiner nichtexpandierten Form hält, so dass die Kraft der Selbstexpansion nicht ausreicht, die Hülle zu dehnen.

2. Kombination nach Anspruch 1, **dadurch gekennzeichnet, dass** der Stent mindestens eine radiale öffnung aufweist.

3. Kombination nach einem der Ansprüche 1 oder 2, **dadurch gekennzeichnet, dass** er über seinen entspannten Zustand hinaus mittels des Ballonkatheters weiter aufweitbar ist.

4. Kombination nach einem der Ansprüche 1 bis 3, **dadurch gekennzeichnet, dass** die Hülle aus einem expandierbaren PTFE besteht.

5. Kombination nach einem der Ansprüche 1 bis 4, **dadurch gekennzeichnet, dass** die Hülle aus einem homologen Material besteht.

6. Kombination nach einem der Ansprüche 1 bis 5, **dadurch gekennzeichnet, dass** die Hülle aus einem tierischen Material besteht.

7. Kombination nach einem der Ansprüche 1 bis 6, **dadurch gekennzeichnet, dass** die Hülle aus Schweine-, Rinder- oder Pferdevenen besteht.

8. Kombination nach einem der Ansprüche 1 bis 7, **dadurch gekennzeichnet, dass** das selbstexpandierbare Material eine Nickel-Titan-Legierung ist.

9. Kombination nach einem der Ansprüche 1 bis 7, **dadurch gekennzeichnet, dass** das selbstexpandierbare Material ein Federstahl ist.

10. Kombination nach einem der Ansprüche 1 bis 7, **dadurch gekennzeichnet, dass** das selbstexpandierbare Material ein polymerer Werkstoff ist.

## Claims

1. Combination comprising a device for implanting a stent graft in a body vessel and a stent graft,
- having a self-expanding hollow cylindrical stent;
- having a sheath which encases the stent in order to form a stent graft; and
- having an expandable balloon for expanding the stent graft following insertion into the body vessel;
**characterized in that**
- the sleeve is fabricated from irreversibly expandable material and keeps the stent in its non-expanded form before the placement in the body vessel, so that the force of the self-expansion is not sufficient to stretch the sheath.

2. Combination according to Claim 1, **characterized in that** the stent has at least one radial opening.

3. Combination according to either of Claims 1 and 2, **characterized in that** it can be expanded further beyond its relaxed state by means of the balloon catheter.

4. Combination according to one of Claims 1 to 3, **characterized in that** the sheath consists of an expandable PTFE.

5. Combination according to one of Claims 1 to 4, **characterized in that** the sheath consists of a homologous material

6. Combination according to one of Claims 1 to 5, **characterized in that** the sheath consists of an animal material.

7. Combination according to one of Claims 1 to 6, **characterized in that** the sheath consists of pig, beef or horse veins.

8. Combination according to one of Claims 1 to 7, **characterized in that** the self-expanding material is a nickel-titanium alloy.

9. Combination according to one of Claims 1 to 7, **characterized in that** the self-expanding material is a spring steel.

10. Combination according to one of Claims 1 to 7, **characterized in that** the self-expanding material is a polymer material.

## Revendications

1. Combinaison constituée d'un dispositif pour implanter un implant dit "Stent » dans un vaisseau corporel et d'un stent
- avec un stent cylindrique creux auto-expansible ;
- avec une gaine qui enveloppe le stent pour former un implant ; et
- avec un ballon expansible pour l'expansion du stent après insertion dans le vaisseau corporel ;
**caractérisée en ce que** :
la gaine est fabriquée dans un matériau expansible de manière irréversible et maintient le stent dans sa forme non expansée avant qu'il ne soit placé dans le vaisseau corporel, en sorte que la force de l'auto-expansion ne suffit pas pour dilater la gaine.

2. Combinaison selon la revendication 1, **caractérisée en ce que** le stent présente au moins une ouverture radiale.

3. Combinaison selon l'une des revendications 1 ou 2, **caractérisée en ce qu'**elle peut être davantage expansée au moyen du cathéter à ballon, au-delà de son état détendu.

4. Combinaison selon l'une des revendications 1 à 3, **caractérisée en ce que** la gaine est constituée d'un PTFE extensible.

5. Combinaison selon l'une des revendications 1 à 4, **caractérisée en ce que** la gaine est constituée d'un matériau homologue.

6. Combinaison selon l'une des revendications 1 à 5, **caractérisée en ce que** la gaine est constituée d'un matériau animal.

7. Combinaison selon l'une des revendications 1 à 6, **caractérisée en ce que** la gaine est constituée de veines de porc, de boeuf ou de cheval.

8. Combinaison selon l'une des revendications 1 à 7, **caractérisée en ce que** le matériau auto-expansible est un alliage de nickel et de titane.

9. Combinaison selon l'une des revendications 1 à 7, **caractérisée en ce que** le matériau auto-expansible est un acier à ressort.

10. Combinaison selon l'une des revendications 1 à 7, **caractérisée en ce que** le matériau auto-expansible est un matériau polymère.
